# FASCICULE DE BREVET EUROPEEN

(11) **EP 0 827 552 B1**
(45) Date de publication et mention de la délivrance du brevet: **02.01.2002**
(21) Numéro de dépôt: 97915516.5
(22) Date de dépôt: 19.03.1997
(51) Int. Cl.: C12Q 1/68

(54) **DETECTION D'UNE SEQUENCE NUCLEOTIDIQUE AVEC AMPLIFICATION DE SIGNAL**
NACHWEIS VON NUKLEOTIDSEQUENZEN DURCH SIGNALAMPLIFIZIERUNG
NUCLEOTIDE SEQUENCE DETECTION WITH SIGNAL AMPLIFICATION

(30) Priorité: 19.03.1996 FR 9603412
(43) Date de publication de la demande: 11.03.1998
(73) Titulaire: BIO MERIEUX, 69280 Marcy l'Etoile (FR)
(72) Inventeur: DELAIR, Thierry, F-69700 Echalas (FR); ELAISSARI, Abdelhamid, F-69007 Lyon (FR); CHARLES, Marie-Hélène, F-69420 Condrieu (FR); MANDRAND, Bernard, F-69100 Villeurbanne (FR)
(74) Mandataire: Tonnellier, Jean-Claude
(86) Numéro de dépôt international: FR9700483
(87) Numéro de publication internationale: WO9735031

(56) Documents cités:
- EP-A- 0 159 719
- EP-A- 0 647 719
- WO-A-94/00600
- WO-A-95/08000

## Description

La présente invention a pour objet un réactif et un procédé pour la détection d'une séquence nucléotidique dans un échantillon.

Il est souvent nécessaire de déterminer si un gène, une partie de gène ou une séquence nucléotidique particulière est présent chez un organisme vivant, dans un extrait cellulaire ou dans un échantillon biologique.

La recherche de séquences nucléotidiques spécifiques est utilisée notamment pour la détection d'organismes pathogènes, la détermination de la présence d'allèles ou la détection de la présence de lésions dans un génome. Des maladies génétiques telles que la maladie de Huntington, la myopathie de Duchenne, la phénylcétonurie et la β-thalassémie peuvent être diagnostiquées par le biais de l'analyse de l'ADN des individus. De plus, le diagnostic ou l'identification de virus, de viroïdes, de bactéries, de champignons ou de parasites peut être réalisé par des expériences d'hybridation avec des sondes nucléiques.

Différents types de méthodes de détection des acides nucléiques sont décrits dans la littérature. Ces méthodes reposent sur les propriétés d'appariement purine-pyrimidine des brins complémentaires d'acides nucléiques dans les duplex ADN-ADN, ADN-ARN et ARN-ARN. Ce processus d'appariement s'effectue par l'établissement de liaisons hydrogène entre les bases adénosinethymine (A-T) et guanosine-cytosine (G-C) de l'ADN double brin. Des paires de bases adénosine-uracile (A-U) peuvent également se former par liaisons hydrogène dans les duplex ADN-ARN ou ARN-ARN. L'appariement de brins d'acides nucléiques pour la détermination de la présence ou de l'absence d'une molécule d'acide nucléique donnée est communément appelée par "hybridation d'acides nucléiques" ou simplement "hybridation".

Sur la base des propriétés des acides nucléiques, des techniques ont été développées permettant de mettre en évidence et de quantifier, dans un échantillon à analyser, un acide nucléique appelé cible. Ces techniques, qui sont bien connues, peuvent être divisées en deux grands groupes : les techniques dites de détection directe telles que celle dite de SOUTHERN et la technique dite "Dot-blot" pour la détection d'ADN ou la technique NORTHERN pour la détection d'ARN, et les techniques dites indirectes telles que la technique sandwich ou "Reverse-Dot".

Une des principales difficultés rencontrées lors de la mise au point d'un test pour détecter une séquence nucléotidique cible d'intérêt est le seuil de sensibilité des méthodes d'hybridation, et diverses méthodes ont été décrites afin d'accroître la puissance de détection de ces techniques d'hybridation. Ces méthodes dites "d'amplification" peuvent intervenir à différents stades d'un procédé de détection par sondes nucléiques. On peut distinguer deux catégories : l'amplification de cible ou de signal.

Les techniques d'amplification de cible sont connues. Un inconvénient de ces techniques réside dans la difficulté à quantifier la cible nucléique d'intérêt après l'étape d'amplification.

D'autres approches, concernant l'amplification de signal, ont été décrites. Ainsi, les brevets US-4 731 325 et EP-0 225 807 décrivent des techniques utilisant une pluralité de sondes de détection pouvant s'hybrider sur la cible. Dans beaucoup de cas (nécessité de différencier des espèces proches en bactériologie ou mise en évidence de maladies génétiques), il n'est pas possible d'utiliser cette technique car une seule séquence spécifique sur la cible est utilisable pour hybrider une sonde de détection.

Certaines techniques décrites consistent à augmenter le nombre des traceurs, c'est-à-dire de molécules capables de générer un signal, directement ou indirectement, sur la sonde de détection. Le traceur peut notamment être une biotine, un fluorophore, une enzyme ou un groupement radioactif. La sonde de détection est greffée à un polymère, qui peut être de nature nucléotidique, sur lequel sont fixés, le plus souvent par covalence, un nombre de traceurs supérieur à deux (voir par exemple US-4 687 732, EP-0 373 956, WO-88/02784, WO-90/00622, EP-0 292 128, WO-89/03849 et EP-0 173 339).

L'un des inconvénients de ces techniques réside dans la nécessité de réaliser un couplage contrôlé entre la sonde et le nombre de traceurs. Ce double couplage n'est pas aisé à maîtriser pour avoir un maximum de traceurs pour une sonde de détection. Des systèmes où le traceur est incorporé de manière contrôlée, par exemple au cours de la synthèse automatique d'oligodésoxyribonucléotides, ont été décrits (Pieles U. et al., Nucleic Acids Research, 18, 4355-4360 (1990) ou Misiura K. et al., Nucleic Acids Research, 18, 4345-4354 (1990)). Mais dans ce cas, le nombre de traceurs incorporés est faible en raison des limitations inhérentes à la synthèse sur support solide. De plus, lorsque le nombre de traceurs augmente, la sonde de détection est facilement masquée par les traceurs et le rendement d'hybridation diminue.

Ces mêmes inconvénients se retrouvent dans le système décrit dans le brevet US-4 882 269. Une sonde dite primaire de nature nucléotidique comportant une queue de type polymère quelconque s'hybride sur la cible. Cette queue peut porter un traceur révélable par une sonde secondaire adaptée. Dans le cas où la queue est de nature nucléotidique, la sonde secondaire est de nature nucléotidique et peut s'hybrider avec la queue. Pour que le système fonctionne, il faut une queue nucléotidique de grande taille, de séquence différente de la sonde primaire, et il faut multiplier les sondes secondaires marquées. Cela est réalisé en pratique par des techniques de biologie moléculaire où la sonde primaire est clonée dans un phage et les sondes secondaires sont complémentaires de différentes séquences du phage.

Un autre système est décrit dans le brevet EP-0 153 873, dans lequel une partie d'une sonde nucléique dite primaire s'hybride avec la cible. Cette sonde primaire comprend une deuxième partie sur laquelle peut s'hybrider une deuxième sonde multimarquée dite secondaire. Dans la réalisation pratique, ces sondes primaires sont fabriquées par des techniques de biologie moléculaire comme le clonage (par exemple clonage d'une séquence spécifique de la cible dans un fragment de phage M13) qui sont peu adaptées à des processus de fabrication à grande échelle.

Le document EP-0 204 510 décrit un procédé dans lequel la cible nucléique est mise en contact avec une première sonde appelée sonde réceptrice, une deuxième sonde appelée sonde amplificatrice et une troisième sonde, appelée sonde marquée, capable de s'hybrider avec la deuxième sonde amplificatrice. La sonde réceptrice qui s'hybride avec la cible possède une queue nucléotidique homopolymère (par exemple polyA). La sonde amplificatrice contient Une séquence complémentaire de la queue (par exemple polyT). La sonde de marquage contient une séquence nucléotidique capable de s'hybrider avec la sonde amplificatrice (par exemple polyA marquée). Cette combinaison de sondes constitue un empilement conduisant à une amplification de signal. Un autre type d'empilement est décrit dans le brevet EP-0 450 594. Dans ces deux cas, les empilements qui se produisent dans le milieu d'hybridation ne sont pas contrôlés et conduisent à une mauvaise reproductibilité et donc à des problèmes de quantification. De plus la multiplication d'étapes successives d'hybridation entraîne des pertes qui entraînent un gain médiocre en amplification de signal.

Dans le brevet FR-2 710 075, on a décrit l'utilisation d'un copolymère sur lequel sont greffées plusieurs unités oligonucléotidiques complémentaires d'une sonde de détection marquée et aussi complémentaires de la cible. Cependant, les polymères ont tendance à former des agrégats nuisant à l'homogénéité des résultats.

Il a par ailleurs été décrit dans la demande de brevet EP-A-0 159 719, une méthode d'hybridation pour la détection d'un matériel génétique cible, utilisant des segments oligonucléotidiques, portés ou non par une même sonde, reconnaissant deux séquences différentes de la cible, et conduisant à la formation de multihybrides. Les segments sont marqués soit par des particules, soit par des entités susceptibles de générer un signal. Dans cette méthode, qui implique généralement la présence d'une seule entité génératrice de signal fonctionnelle par molécule cible, et qui ne permet donc généralement pas l'amplification de signal, les particules permettent la capture de la cible et son isolement soit par sédimentation ou filtration, soit par agglutination et précipitation dans le cas où les segments sont portés par des sondes distinctes ou non, lesdites sondes étant chacune fixées en plusieurs exemplaires sur une particule.

On a maintenant trouvé un système de détection ne présentant pas les inconvénients précités.

L'invention a pour objet un nécessaire (encore appelé "kit") pour la détection d'une séquence nucléotidique d'intérêt à l'aide d'une sonde nucléotidique marquée, avec amplification de signal, caractérisé par le fait qu'il contient, dans des conteneurs appropriés, ladite sonde nucléotidique, marquée avec un traceur, et un réactif comprenant essentiellement une suspension de particules sur lesquelles est immobilisée au moins une série d'unités oligonucléotidiques, chacune desdites unités oligonucléotidiques de ladite série, toutes identiques, comprenant, au moins, une séquence nucléotidique capable d'hybridation avec ladite séquence d'intérêt et une séquence nucléotidique capable d'hybridation avec ladite sonde, ledit réactif contenant plus de 10, et en particulier plus de 50, desdites unités oligonucléotidiques de ladite série par particule.

Bien entendu, le nombre d'unités nucléotidiques donné ici est un nombre moyen d'unités nucléotidiques par particule.

Par exemple, le réactif utilisé dans le kit de l'invention peut comprendre, en moyenne, de 100 à 1000 desdites unités oligonucléotidiques d'une même série par particule.

La suspension de particules est également appelée "latex". On appelle ici latex une suspension de particules de polymère, naturel ou synthétique, dans un milieu liquide, notamment un milieu aqueux, les particules étant insolubles dans ledit milieu et ayant des dimensions inférieures à 10 µm.

Les particules du latex utilisables dans la réalisation du réactif peuvent être des produits commerciaux ou peuvent être préparées de façon connue, notamment par polymérisation en émulsion, en suspension, en dispersion, ou par précipitation ; voir par exemple R. Arshady, Colloid Polym. Sci. 270:717-732 (1992) et le brevet PR-2 676 451. Ces particules peuvent porter des fonctions réactives telles que par exemple des groupements thiol, amine ou carbonyle (notamment aldéhyde) ; des groupements acide carboxylique, des groupements acide carboxylique activés, notamment sous forme de chlorure d'acide, d'anhydride, d'anhydride mixte, d'ester (ester de N-hydroxysuccinimide, ester de p-nitrophényle, etc.) ; des groupements hydroxyle et leurs dérivés (tosylates, mésylates, etc.) ; des groupements halogénés ; des doubles liaisons activées (par exemple divinylsulfone ; des groupements carbonyle α,β-insaturés, etc.). Le matériau polymère constituant les particules peut être issu notamment de monomères choisis parmi les dérivés de type vinylique tels que le styrène, les dérivés acryliques (acides acrylique et méthacrylique, acrylamides et méthacrylamides, esters acryliques et méthacryliques), les alcools, esters et éthers vinyliques, etc.).

Les particules, qui sont de préférence de taille homogène, ont par exemple des dimensions pouvant aller de 50 nm à 5 µm, et en particulier de 100 à 1000 nm.

Le terme "unité oligonucléotidique" tel qu'utilisé dans la présente demande, désigne un oligonucléotide constitué par un enchaînement d'au moins 5 désoxyribonucléotides ou ribonucléotides comprenant éventuellement au moins un nucléotide modifié, par exemple au moins un nucléotide comportant une base modifiée telle que l'inosine, la méthyl-5-désoxycytidine, la diméthylamino-5-désoxyuridine, la désoxyuridine, la diamino-2,6-purine, la bromo-S-désoxyuridine ou toute autre base modifiée permettant l'hybridation. Cet oligonucléotide peut aussi être modifié au niveau de la liaison internucléotidique comme par exemple les phosphorothioates, les H-phosphonates, les alkyl-phosphonates, au niveau du squelette comme par exemple les α-oligonucléotides (FR-2 607 507) ou les PNA (M. Egholm et al., J. Am. Chem. Soc., (1992), 114, 1895-1897). Ces diverses modifications peuvent éventuellement être prises en combinaison.

La séquence d'intérêt est notamment une séquence nucléotidique simple brin, éventuellement obtenue par dénaturation préalable d'un hybride (ADN-ADN, ADN-ARN ou ARN-ARN) selon les techniques classiques (physiques, chimiques ou enzymatiques). Le système d'amplification de signal de l'invention peut également servir à la détection d'une séquence double brin, en opérant selon la technique de la triple hélice à laquelle il peut s'appliquer.

Les traceurs utilisés pour marquer la sonde de détection sont choisis parmi les traceurs usuels. Ce sont par exemple :
- des enzymes qui sont aptes à produire un signal détectable par exemple par colorimétrie, fluorescence, ou luminescence, comme la peroxydase de raifort, la phosphatase alcaline, la β-galactosidase, la glucose-6-phosphate déshydrogénase, etc. ;
- des chromophores comme les composés fluorescents, luminescents, colorants ;
- des groupements à densité électronique détectable par microscopie électronique ou par leurs propriétés électriques comme la conductivité, l'ampérométrie, la voltamétrie, ou les mesures d'impédance ;
- des groupements détectables par des méthodes optiques (comme la diffraction, la résonance plasmon de surface, la variation d'angle de contact) ou par des méthodes physiques comme la spectroscopie de force atomique, l'effet tunnel ;
- des molécules radioactives comme le ³²P, le ³⁵S ou le ¹²⁵I.

Les méthodes covalentes ou non covalentes pour attacher ces traceurs dépendent du type de traceur et sont bien connues.

Des systèmes indirects peuvent aussi être utilisés, par exemple, ceux comprenant les haptènes, détectables par un anticorps spécifique, ou une protéine comme le couple biotine-avidine ou biotine-streptavidine, ou encore un couple sucre/lectine. Dans ce cas c'est l'anticorps ou la protéine qui porte un traceur.

Les acides nucléiques hybrides de type ADN/ADN, ARN/ARN, ADN/ARN, peuvent être détectés par des anticorps antihybrides, ou par des protéines spécifiques comme les polymérases phagiques.

Si la sonde de détection est constituée de nucléotides modifiés comme les nucléotides d'anomérie alpha ou les PNA (P.E NIELSEN et al., Science, 254,1497-1500 (1991)), des anticorps anti-alphanucléotides ou anti-PNA peuvent être utilisés.

Selon un premier mode de réalisation, le réactif utilisé selon l'invention est caractérisé par le fait que, dans une même unité oligonucléotidique, lesdites séquences capables d'hybridation sont distinctes et non chevauchantes. Dans un tel cas, la séquence de la sonde marquée peut être choisie arbitrairement, et ladite séquence oligonucléotidique capable d'hybridation avec la sonde est alors une séquence complémentaire de celle de la sonde.

Dans un système utilisant un tel réactif, la sonde marquée peut donc être utilisée comme sonde de détection universelle. En outre, un tel système convient plus particulièrement pour effectuer la détection en une seule étape, notamment sans lavage(s) intermédiaire(s).

Dans un autre mode de réalisation, le réactif utilisé selon l'invention est caractérisé par le fait que dans une même unité oligonucléotidique, l'une desdites séquences capables d'hybridation est incluse dans l'autre. Autrement dit, l'une desdites séquences est constituée par une partie de l'autre séquence, ou bien, à la limite, les deux séquences sont confondues (elles sont identiques) et, en fait, n'en font qu'une, dans l'unité oligonucléotidique. Dans ce mode de réalisation, la sonde marquée doit évidemment être adaptée à la cible (séquence d'intérêt) : ou bien la sonde est identique à la séquence d'intérêt, ou bien la sonde doit au moins être homologue de la séquence d'intérêt, c'est-à-dire être capable de s'hybrider avec la séquence complémentaire de la séquence d'intérêt.

Généralement, le réactif utilisé selon l'invention contient quelques centaines d'unités oligonucléotidiques d'une même série (toutes identiques), notamment de 100 à 1000 unités oligonucléotidiques, telles que définies ci-dessus, par particule. Ce réactif est également appelé ici "conjugué de détection".

Les unités oligonucléotidiques présentes dans le réactif d'amplification de l'invention sont généralement des oligonucléotides ayant une longueur de 5 à 100 nucléotides et en particulier de 10 à 40 nucléotides.

Les unités oligonucléotidiques sont immobilisées sur les particules du latex selon les méthodes connues, par exemple par adsorption, par interaction hydrophobe, par interaction ionique, ou par établissement de liaisons hydrogène, ou encore par couplage de façon à établir une liaison covalente avec les particules, éventuellement par l'intermédiaire d'un bras espaceur.

Le couplage d'unités oligonucléotidiques avec les particules du latex peut être effectué selon l'une des méthodes suivantes. Par exemple, dans le cas d'une méthode directe, on synthétise un oligonucléotide ayant une fonction réactive à un endroit quelconque de la chaîne nucléotidique, comme par exemple à l'extrémité 5' ou à l'extrémité 3', ou sur une base nucléotidique ou sur un phosphate internucléotidique, ou encore sur la position 2' du sucre nucléotidique. L'oligonucléotide est ensuite couplé aux particules du latex préparées au préalable et comportant une fonction réactive complémentaire de celle de l'oligonucléotide, c'est-à-dire telle que la réaction des deux fonctions réactives entre elles permet l'établissement d'une liaison covalente entre l'oligonucléotide et une particule. A titre d'exemples de couples de fonctions réactives, on peut coupler des amines primaires avec un acide carboxylique activé, un aldéhyde, un isocyanate, un isothiocyanate ou une double liaison activée par au moins un carbonyle, par une sulfone, par un groupement sulfoxyde ou par un nitrile ; ou bien une fonction thiol avec un halogénoalkyle ou avec un dérivé à double liaison carbone-carbone activée.

Dans une méthode de couplage indirect, l'oligonucléotide et les particules sont chacun porteurs d'une fonction réactive, ces fonctions réactives pouvant être identiques ou différentes l'une de l'autre, ces deux fonctions n'étant pas complémentaires, mais étant capables de réagir avec un agent intermédiaire de couplage qui est un réactif bifonctionnel pouvant éventuellement joue le rôle de bras espaceur. Cet agent est dit homobifonctionnel si les deux fonctions sont identiques et hétérobifonctionnel si les deux fonctions sont différentes. Parmi les agents de couplage homobifonctionnels, on peut citer le DITC (phénylène-1,4-diisothiocyanate), le DSS (disuc-cinimidylsubérate) ou analogues lorsque les deux fonctions réactives sont des fonctions amines primaires. Parmi les agents de couplage hétérobifonctionnels, on peut citer le SMCC (succinimidyl-4-(N-maléimidométhyl)cyclohexane-1-carboxylate) lorsque les deux fonctions réactives présentent chacune indépendamment de l'autre une fonction amine primaire et une fonction thiol, le SMPB (succinimi-dyl-4-(p-maléimido phényl) butyrate) ou analogues.

Après le couplage de l'oligonucléotide avec les particules, l'excès éventuel de fonctions réactives sur les particules peut être neutralisé, le cas échéant, de façon connue en soi. Par exemple, les groupements aldéhyde en excès peuvent être neutralisés par une amine primaire telle que l'éthanolamine (et inversement), les groupements maléimide peuvent être neutralisés par un thiol (tel que la thioéthanolamine ou le dithiothréitol), etc.

On peut immobiliser de la même façon, sur des particules couplées à une première série d'unités oligonucléotidiques toutes identiques, au moins une seconde série d'unités oligonucléotidiques toutes identiques mais différentes de celles de la première série. Par exemple, les unités oligonucléotidiques de la seconde série comprennent une séquence nucléotidique capable d'hybridation avec une seconde séquence d'intérêt différente de la séquence d'intérêt reconnue par les unités oligonucléotidiques de la première série.

L'invention concerne également l'utilisation d'un réactif, constitué par le conjugué de détection tel que défini précédemment, comme réactif d'amplification de signal dans un procédé de détection d'une séquence nucléotidique d'intérêt.

La séquence d'intérêt est soit une séquence de la substance cible (celle que l'on veut détecter effectivement), soit une séquence liée à la substance cible, soit encore (dans des méthodes de compétition) une séquence liée à un analogue de la substance-cible, compétiteur de celle-ci.

L'invention concerne notamment un procédé de détection, avec amplification de signal d'une séquence nucléotidique d'intérêt susceptible d'être immobilisée sur un support solide, dans lequel on utilise une sonde nucléotidique de détection marquée avec un traceur, caractérisé par le fait que l'on ajoute à un milieu liquide en contact avec ledit support solide, dans des conditions permettant l'hybridation :
- un réactif tel que défini ci-dessus,
- et la sonde de détection marquée,
puis on révèle selon les méthodes usuelles la présence éventuelle du traceur immobilisé sur le support solide.

Le terme "support solide" tel qu'utilisé ici inclut tous les matériaux sur lesquels peut être immobilisé un oligonucléotide pour une utilisation dans des tests diagnostiques, en chromatographie d'affinité et dans des processus de séparation. Des matériaux naturels ou de synthèse, modifiés ou non chimiquement peuvent être utilisés comme support solide, notamment les polysaccharides tels que les matériaux à base de cellulose, par exemple du papier, des dérivés de cellulose tels que l'acétate de cellulose et la nitrocellulose, du dextran ; des polymères tels que polychlorures de vinyle, polyéthylènes, polystyrènes, polyacrylates, polyamides, ou copolymères à base de monomères vinyl aromatiques, alkylesters d'acides α-β insaturés, esters d'acides carboxyliques insaturés, chlorure de vinylidène, diènes ou composés présentant des fonctions nitrile (comme acrylonitrile) ; des polymères de chlorure de vinyle et de propylène ; des polymères de chlorure de vinyle et d'acétate de vinyle ; des copolymères à base de styrènes ou dérivés substitués du styrène ; des fibres naturelles telles que le coton et des fibres synthétiques telles que le nylon ; des matériaux inorganiques tels que la silice, le verre, une céramique, le quartz. Le choix d'un matériau de support, dans chaque cas particulier, peut être effectué à partir de simples expériences de routine.

Le support solide utilisé dans la présente invention est notamment un polymère de polystyrène, un copolymère butadiène-styrène ou un copolymère butadiène-styrène en mélange avec un ou plusieurs polymères ou copolymères choisis parmi le polystyrène, les copolymères styrène-acrylonitrile ou styrène-méthylméthacrylate de méthyle, les polypropylènes, les polycarbonates ou analogues. Le support solide utilisé est notamment un polystyrène ou un copolymère à base de styrène comprenant entre environ 10 et 90 % en poids de motifs styrène.

Le support solide utilisé peut être, sous les formes usuelles appropriées, par exemple, sous la forme d'une plaque de microtitration, d'une feuille, d'un cône, d'un tube, d'un puits, de billes ou analogues.

Le procédé de l'invention peut servir à la détection et/ou au dosage d'un fragment d'acide nucléique cible susceptible d'être présent dans un échantillon, notamment dans les cas déjà mentionnés dans l'introduction de la présente demande : diagnostic de maladies génétiques, identifications d'agents pathogènes tels que bactéries, virus, champignons, ou parasites, etc. La séquence nucléotidique d'intérêt est une partie déterminée de la séquence nucléotidique de la cible, qui peut être choisie, notamment, comme moyen de caractérisation d'une espèce, d'un genre, d'un allèle ou analogues. La séquence nucléotidique d'intérêt est fixée sur le support solide directement ou indirectement par l'intermédiaire d'un ligand. Le ligand peut notamment être une sonde de capture choisie pour être complémentaire d'une autre région de la cible, selon la technique sandwich, qui est bien connue. Dans un autre mode de réalisation de la technique sandwich, la sonde de capture est elle-même greffée sur un copolymère immobilisée ou immobilisable sur le support solide, par exemple par adsorption passive, c'est-à-dire sans formation d'une liaison covalente entre le support et le copolymère portant la sonde de capture. Cette méthode peut contribuer à réduire le bruit de fond. La séquence d'intérêt peut également être fixée directement sur le support solide par l'intermédiaire de la cible selon la technique "Reverse-Dot".

Le procédé de l'invention est également applicable aux immunoessais et notamment à la détection d'haptènes, d'antigènes, de polypeptides ou d'anticorps, dans des processus avec ou sans compétition. Par exemple, un oligonucléotide peut être greffé, par liaison covalente de préférence, à un ligand susceptible de réagir spécifiquement avec une molécule cible. Cet oligonucléotide joue alors le rôle de la "séquence d'intérêt". Dans les techniques de compétition, l'oligonucléotide peut être greffé, de préférence par covalence, à un ligand, ledit ligand étant susceptible d'entrer en compétition avec la cible pour sa fixation à un anti-ligand spécifique. Les techniques de couplage entre un ligand et l'oligonucléotide dépendent de la nature du ligand et sont bien connues. Il est évidemment nécessaire de choisir une méthode de couplage qui n'altère pas la capacité du ligand à reconnaître l'anti-ligand, ce qui peut être facilement vérifié par de simples expériences de routine.

Le terme "anticorps" désigne notamment les anticorps monoclonaux ou polyclonaux, les fragments d'anticorps et les anticorps obtenus par recombinaison génétique.

Le terme "haptène" désigne une molécule de taille insuffisante pour être immunogène, mais qui par couplage avec une protéine, par exemple, permet par immunisation d'animaux l'obtention d'anticorps reconnaissant ladite molécule.

A titre illustratif, si la cible à doser est un antigène, la séquence d'intérêt (c'est-à-dire l'oligonucléotide) est greffée sur un anticorps spécifique de l'antigène. Cet anticorps, après réaction avec l'antigène, peut réagir par l'intermédiaire de la séquence d'intérêt pour former un complexe avec le réactif oligonucléotides-latex. L'unité oligonucléotidique greffée sur les particules de latex comprend une séquence complémentaire d'au moins une partie de la séquence d'intérêt. Avec une sonde de détection marquée, on peut ainsi révéler l'antigène avec une bonne sensibilité, grâce à l'amplification du signal.

Si l'unité oligonucléotidique du réactif comprend une séquence spécifique de la séquence d'intérêt et une autre séquence, cette autre séquence sera complémentaire de la séquence, arbitraire, de la sonde. Une réduction du nombre d'étapes est possible en utilisant un tampon approprié permettant à la fois la réaction du ligand sur l'anti-ligand et l'hybridation des séquences nucléotidiques entre elles. Un essai en une étape est réalisable, l'anticorps étant greffé à la séquence d'intérêt, le réactif de détection latex-oligonucléotides et la sonde de détection réagissant pendant la même incubation.

Dans le dessin annexé, la figure unique est un graphe représentant la variation du signal avec amplification (-◆-) et sans amplification (-□-) dans l'essai décrit à l'exemple 2 ci-après. En abscisse est portée la quantité de copies d'ADN cible, exprimée en puissances de 10, et en ordonnée le signal exprimé en unités relatives de fluorescence (URF).

Les exemples suivants illustrent l'invention. Dans ces exemples, les séquences nucléotidiques utilisées sont les suivantes :

- séquence 3059 :
TCAATCTCGGGAATCTCAATGTTAG

- séquence 2908 :
AACGCTACTACTATTAG

- séquence 3057 :
CTACTAATAGTAGTAGCGTT

### EXEMPLES

### EXEMPLE 1 : Couplage d'un l'oligonucléotide avec des particules de latex

On effectue préalablement une activation de l'oligonucléotide 2908 avec du phénylène-diisothiocyanate dans les conditions suivantes : 30 nanomoles d'oligonucléotide sont mis en solution dans 25 µl de tampon borate de sodium 0,1 M pH 9,3 auquel on ajoute 60 µl d'une solution de phénylène-diisothiocyanate (DITC) dans le diméthyl-formamide à 30 g/L. Après 2 heures d'incubation à 37°C, on extrait par le butanol le DITC n'ayant pas réagi, puis on recueille la phase aqueuse et l'évapore à sec sous pression réduite. On obtient ainsi l'oligonucléotide 2908 activé.

Dans un tampon carbonate de sodium 0,1 M, pH 9,3 contenant l'oligonucléotide 2908 activé, on ajoute un latex, de façon à obtenir une suspension contenant 1 % en poids de particules et 100 µg d'oligonucléotide 2908 par ml.

Le latex a été obtenu de façon analogue à celle décrite dans l'exemple 1 du brevet FR-2 676 451, au départ d'un mélange de styrène et de chlorhydrate d'aminométhyl styrène, contenant 1 % en poids de ce dernier (qui est lui-même un mélange 60:40, en moles, des isomères para et méta). Les particules ont des dimensions moyennes de l'ordre de 430 nm.

Après 15 heures sous agitation, on soumet à une centrifugation et le culot de centrifugation est repris dans un tampon glycine 0,1 M NaCl 0,15 M, pH 8,2.

Le rendement de couplage est déterminé en dosant (en UV à 280 nm) la quantité d'oligonucléotides résiduels dans le surnageant après centrifugation du latex. Ce rendement est de l'ordre de 70 %.

### EXEMPLE 2 :

Détection d'un fragment d'acide nucléique du virus de l'hépatite B par protocole sandwich sur automate VIDAS en utilisant pour la détection des oligonucléotides couplés à des particules de latex, obtenus à l'exemple 1 ci-dessus.

Le protocole suivant est effectué automatiquement sur l'automate VIDAS® commercialisé par la société bioMérieux-Vitek.

La réaction est conduite dans un support conique appelé SPR ("Solid Phase Receptacle"), réalisé à partir d'un matériau vendu sous la dénomination "K résine" (copolymère butadiène-styrène) et commercialisé par la société bioMérieux-Vitek (USA). Les divers réactifs sont disposés dans les différents puits d'une barrette et les étapes successives se déroulent dans le SPR qui fait office de pipette. La réaction d'hybridation sandwich décrite dans le protocole ci-dessous se produit sur la paroi interne du cône.

Sur la surface interne du SPR, est fixé passivement oligonucléotide 3059 (spécifique de l'ADN du virus de l'hépatite B) couplé à un copolymère (décrit à l'exemple 1 de FR-2 710 075). La concentration utilisée est de 0,15 nmole/ml d'oligonucléotides dans un volume de 300 µL d'une solution de PBS 4x (phosphate de Sodium 200 mM pH 7,0, NaCl 600 mM). Après 18 heures à température ambiante, ou deux heures à 37°C, les cônes sont lavés 2 fois par une solution de PBS Tween , puis séchés sous vide.

La barrette contient l'ensemble des réactifs nécessaires à la détection, dans des puits indépendants, c'est-à-dire :
- puits 2 : 100 µl d'une solution à 0,015 nmoles/ml oligonucléotide 2908 lié aux particules de latex, dans un tampon PEG (phosphate de sodium 150 mM, NaCl 450 mM, pH 7 + ADN de sperme de saumon 0,14 mg/ml (Sigma D 9156) + 20 g/l de PEG 4000 (Merck 807490) + 6,5 g/l de Tween 20 (Biorad 170-6531)),
- puits 3 : 200 µl d'une solution à 0,045 nmoles/ml dans le tampon PEG de la sonde de détection. La sonde de détection est l'oligonucléotide 3057, couplé à la phosphatase alcaline comme décrit dans WO-91/19812.
- puits 4 et 5 : 2 fois 600 µl de solution de lavage PBS Tween,
- puits 6 : 300 µl de substrat MUP (méthyl-4 umbelliféryl phosphate) en solution dans un tampon diéthanolamine.

Dans le premier puits (puits 1) de la barrette, sont déposés 200 µl de la solution de cible dans un tampon PEG. Après mélange, reconstitué dans le puits 2, de la cible (110 µl du puits 1), de la sonde de détection et du conjugué oligonucléotide-latex pour l'amplification (110 µl du puits 3), et incubation pendant 45 minutes du cône contenant ledit mélange, le cône est lavé 2 fois par une solution de PBS Tween. 250 µl de substrat sont aspirés dans le cône pendant 15 minutes, puis relâchés dans une cuvette de lecture. L'appareil mesure le signal fluorescent exprimé en URF (unités relatives de fluorescence).

A titre de comparaison, on a réalisé un essai analogue, mais en omettant l'addition du conjugué 2908-latex dans le puits n° 2.

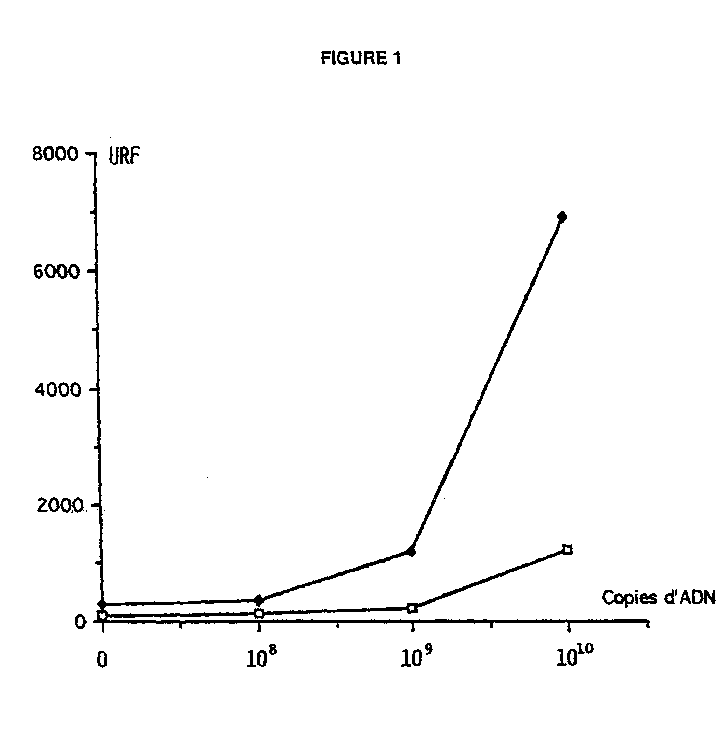

Les résultats sont représentés à la figure 1.

## Revendications

1. Nécessaire pour la détection d'une séquence nucléotidique d'intérêt à l'aide d'une sonde nucléotidique marquée, avec amplification de signal, **caractérisé par le fait qu'**il contient, dans des conteneurs appropriés, ladite sonde nucléotidique, marquée avec un traceur, et un réactif comprenant essentiellement une suspension de particules sur lesquelles est immobilisée au moins une série d'unités oligonucléotidiques, chacune desdites unités oligonucléotidiques de ladite série, toutes identiques, comprenant, au moins, une séquence nucléotidique capable d'hybridation avec ladite séquence d'intérêt et une séquence nucléotidique capable d'hybridation avec ladite sonde, ledit réactif contenant plus de 10 desdites unités oligonucléotidiques de ladite série par particule.

2. Nécessaire selon la revendication 1, **caractérisé par le fait que**, dans une même unité oligonucléotidique, lesdites séquences capables d'hybridation sont distinctes et non chevauchantes.

3. Nécessaire selon la revendication 1, **caractérisé par le fait que**, dans une même unité oligonucléotidique, l'une desdites séquences capables d'hybridation est incluse dans l'autre.

4. Nécessaire selon la revendication 3, **caractérisé par le fait que**, dans une même unité oligonucléotidique, lesdites séquences capables d'hybridation sont identiques.

5. Nécessaire selon l'une quelconque des revendications précédentes, **caractérisé par le fait que** ledit réactif contient en moyenne de 100 à 1000 desdites unités oligonucléotidiques par particule.

6. Nécessaire selon l'une quelconque des revendications précédentes, **caractérisé par le fait que** lesdites particules ont des dimensions inférieures à 10 µm.

7. Nécessaire selon la revendication 6, **caractérisé par le fait que** lesdites particules ont des dimensions pouvant aller de 50 nm à 5 µm et en particulier de 100 nm à 1000 nm.

8. Procédé de détection, avec amplification de signal, d'une séquence nucléotidique d'intérêt susceptible d'être immobilisée sur un support solide, dans lequel on utilise une sonde nucléotidique de détection marquée avec un traceur, **caractérisé par le fait que** l'on ajoute à un milieu liquide en contact avec ledit support solide, dans des conditions permettant l'hybridation :
- un réactif tel que défini dans l'une quelconque des revendications précédentes,
- et la sonde de détection marquée, puis on révèle la présence éventuelle du traceur immobilisé sur le support solide.

9. Procédé selon la revendication précédente, **caractérisé par le fait que** l'on ajoute le réactif et la sonde simultanément, c'est-à-dire sans étape de lavage intermédiaire.

10. Utilisation d'un réactif tel que défini dans l'une quelconque des revendications 1 à 7, comme réactif d'amplification de signal dans un procédé de détection d'une séquence nucléotidique d'intérêt.

## Patentansprüche

1. Kitt zur Detektion einer interessierenden Nukleotidsequenz mit Hilfe einer markierten Nukleotidsonde mit Signalamplifikation, **dadurch gekennzeichnet, dass** er in geeigneten Behältern die mit einem Tracer markierte Nukleotidsonde und ein Reagenz enthält, umfassend im Wesentlichen eine Suspension von Teilchen, auf denen mindestens eine Serie von Oligonukleotideinheiten immobilisiert ist, wobei alle diese Oligonukleotideinheiten der besagten Serie, alle identisch, mindestens eine Nukleotidsequenz, die mit der interessierenden Sequenz hybridisieren kann, und eine Nukleotidsequenz enthalten, die mit der Sonde hybridisieren kann, wobei das Reagenz mehr als zehn der Oligonukleotideinheiten der Serie pro Teilchen enthält.

2. Kitt gemäß Anspruch 1, **dadurch gekennzeichnet, dass** in ein und derselben Obligonukleotideinheit die zur Hybridisierung befähigten Sequenzen voneinander verschieden und nicht überlappend sind.

3. Kitt gemäß Anspruch 1, **dadurch gekennzeichnet, dass** in ein und derselben Oligonukleotideinheit eine der zur Hybridisierung befähigten Sequenzen in der anderen eingeschlossen ist.

4. Kitt gemäß Anspruch 3, **dadurch gekennzeichnet, dass** in ein und derselben Oligonukleotikeinheit die zur Hybridisierung befähigten Sequenzen identisch sind.

5. Kitt gemäß einem der vorstehenden Ansprüche, **dadurch gekennzeichnet, dass** das Reagenz im Mittel 100 bis 1000 der Oligonukleotideinheiten pro Teilchen enthält.

6. Kitt gemäß einem der vorstehenden Ansprüche, **dadurch gekennzeichnet, dass** die Teilchen Abmessungen unterhalb von 10 µm aufweisen.

7. Kitt gemäß Anspruch 6, **dadurch gekennzeichnet, dass** die Teilchen Dimensionen aufweisen, die von 50 nm bis 5 µm und insbesondere von 100 nm bis 1000 nm gehen können.

8. Verfahren zur Detektion mit Signalamplifikation einer interessierenden Nukleotidsequenz, die auf einem festen Träger immobilisiert werden kann, worin man eine mit einem Tracer markierte Nukleotiddetektionssonde nutzt, **dadurch gekennzeichnet, dass** man zu einem flüssigen Milieu in Kontakt mit dem festen Träger unter Bedingungen, die die Hybridisierung gestatten, hinzugibt:
- ein Reagenz, wie es in einem der vorstehenden Ansprüche definiert ist;
- und die markierte Nachweissonde, und anschließend gegebenenfalls die Anwesenheit des auf dem festen Träger immobilisierten Tracers bestimmt.

9. Verfahren gemäß dem vorstehenden Anspruch, **dadurch gekennzeichnet, dass** man das Reagenz und die Sonde gleichzeitig, d. h. ohne zwischengeschalteten Waschschritt hinzugibt.

10. Verwendung eines Reagenz, wie es in einem der Ansprüche 1 bis 7 definiert ist, als Reagenz der Signalamplifikation in einem Detektionsverfahren einer interessierenden Nukleotidsequenz

## Claims

1. Kit for the detection of a nucleotide sequence of interest with the aid of a labelled nucleotide probe, with signal amplification, **characterized in that** it contains, in appropriate containers, the said nucleotide probe, labelled with a marker, and a reagent essentially comprising a suspension of particles on which is immobilized at least one series of oligonucleotide units, each of the said oligonucleotide units of the said series, all identical, comprising, at least, a nucleotide sequence capable of hybridizing with the said sequence of interest and a nucleotide sequence capable of hybridizing with the said probe, the said reagent containing more than 10 of the said oligonucleotide units of the said series per particle.

2. Kit according to Claim 1, **characterized in that**, in the same oligonucleotide unit, the said sequences capable of hybridization are distinct and nonoverlapping.

3. Kit according to Claim 1, **characterized in that**, in the same oligonucleotide unit, one of the said sequences capable of hybridization is nested in the other.

4. Kit according to Claim 3, **characterized in that**, in the same oligonucleotide unit, the said sequences capable of hybridization are identical.

5. Kit according to any one of the preceding claims, **characterized in that** the said reagent contains on average from 100 to 1000 of the said oligonucleotide units per particle.

6. Kit according to any one of the preceding claims, **characterized in that** the said particles have sizes of less than 10 µm.

7. Kit according to Claim 6, **characterized in that** the said particles have sizes which may range from 50 nm to 5 µm and in particular from 100 nm to 1000 nm.

8. Process for the detection, with signal amplification, of a nucleotide sequence of interest capable of being immobilized on a solid support, in which a detection nucleotide probe labelled with a marker is used, **characterized in that** there are added to a liquid medium in contact with the said solid support, under conditions allowing the hybridization:
- a reagent as defined in any one of the preceding claims,
- and the labelled detection probe,
and the possible presence of the marker immobilized on the solid support is then revealed.

9. Process according to the preceding claim, **characterized in that** the reagent and the probe are added simultaneously, that is to say without an intermediate washing step.

10. Use of a reagent as defined in any one of Claims 1 to 7, as signal amplification reagent in a process for the detection of a nucleotide sequence of interest.
